# EUROPÄISCHE PATENTSCHRIFT

(11) **EP 2 454 232 B1**
(45) Veröffentlichungstag und Bekanntmachung des Hinweises auf die Patenterteilung: **14.06.2017**
(21) Anmeldenummer: 10735197.5
(22) Anmeldetag: 06.07.2010
(51) Int. Cl.: C07C 263/10, C07C 265/14

(54) **VERFAHREN ZUR HERSTELLUNG VON ISOCYANATEN IN DER GASPHASE**
METHOD FOR PRODUCING ISOCYANATES IN THE GASEOUS PHASE
PROCÉDÉ DE PRODUCTION D'ISOCYANATES EN PHASE GAZEUSE

(30) Priorität: 17.07.2009 DE 102009033639
(43) Veröffentlichungstag der Anmeldung: 23.05.2012
(73) Patentinhaber: Covestro Deutschland AG, 51373 Leverkusen (DE)
(72) Erfinder: BRUNS, Rainer, 51373 Leverkusen (DE); LORENZ, Wolfgang, 41540 Dormagen (DE); RAUSCH, Andreas Karl, 41564 Kaarst (DE)
(74) Vertreter: Levpat
(86) Internationale Anmeldenummer: PCT/EP2010/004109
(87) Internationale Veröffentlichungsnummer: WO 2011/006609

(56) Entgegenhaltungen:
- EP-A1- 1 935 876
- EP-A2- 1 754 698
- WO-A1-2010/100221

## Beschreibung

Die vorliegende Erfindung betrifft ein Verfahren zur Herstellung von Isocyanaten aus Aminen und Phosgen in der Gasphase.

Isocyanate und insbesondere Diisocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen. Die Umsetzung der Amine mit dem Phosgen kann in der Flüssigphase, in der Gasphase oder durch Phosgenierung eines durch Zerstäubung hergestellten Aminsprays erfolgen.

Die vorliegend Erfindung betrifft ausschließlich die Phosgenierung in der Gasphase.

Diese Verfahrensführung zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Reaktionsbedingungen gasförmig sind. Vorteile der Gasphasenphosgenierung sind unter anderem ein verringerter Phosgen-Hold-Up, die Vermeidung schwer phosgenierbarer Zwischenprodukte sowie erhöhte Reaktionsausbeuten.

Es sind aus dem Stand der Technik verschiedene Verfahren zur Herstellung von Diisocyanaten durch Umsetzung von Diaminen mit Phosgen in der Gasphase bekannt.

EP 289 840 B1 offenbart erstmals ein Verfahren zur Herstellung aliphatischer Diisocyanate durch Phosgenierung der entsprechenden Diamine in der Gasphase, in welchem die dampfförmigen Diamine, gegebenenfalls verdünnt mit einem Inertgas oder mit den Dämpfen eines inerten Lösungsmittels, und Phosgen getrennt voneinander auf Temperaturen von 200 bis 600°C erhitzt und kontinuierlich in einem auf 200 bis 600°C erhitzten Reaktionsraum miteinander zur Reaktion gebracht werden. Dabei liegt der absolute Druck in den Zuleitungen zum Reaktionsraum bei 200 bis 3000 mbar und am Ausgang aus dem Reaktionsraum bei 150 bis 2000 mbar. Die Schrift offenbart nicht, unter welchem Druck die Verdampfung der Diamine durchgeführt wird und innerhalb welcher Zeit sie dem Reaktionsraum zugeführt werden.

EP 593 334 B1 beschreibt erstmals ein Verfahren zur Herstellung aromatischer Diisocyanate in der Gasphase. Gemäß dieser Erfindung variiert die Temperatur, welche in dem Reaktor herrscht, in dem die Phosgenierungsreaktion bewirkt wird, zwischen 250 und 500°C. Dabei ist die Vorerhitzungstemperatur der Reaktionsteilnehmer von derselben Größenordnung wie diejenige, welche zur Durchführung der Phosgenierung erforderlich ist. Der im Reaktor herrschende Druck liegt zwischen 0,5 und 1,5 bar. Aussagen unter welchem Druck die Verdampfung der Diamine durchgeführt wird und innerhalb welcher Zeit sie dem Reaktionsraum zugeführt werden, werden nicht getroffen.

EP 570 799 B1 offenbart ein Verfahren zur Herstellung von aromatischen Diisocyanaten durch Umsetzung entsprechender Diamine mit Phosgen in der Gasphase, dadurch gekennzeichnet, dass Phosgen und Diamin oberhalb des Siedepunkts des Diamins innerhalb einer mittleren Verweilzeit von 0,5 s bis 5 s umgesetzt werden. Dazu werden die dampfförmigen Diamine, gegebenenfalls verdünnt mit einem Inertgas oder mit den Dämpfen eines inerten Lösungsmittels, und Phosgen getrennt auf Temperaturen von 200 bis 600°C erhitzt und kontinuierlich vermischt. Dabei liegt der absolute Druck in den Zuleitungen zum Reaktionsraum bei 200 - 3000 mbar und hinter der Kondensationsstufe des Reaktors bei 150 - 2000 mbar, wobei nach Lehre dieser Schrift die Aufrechterhaltung dieses Differenzdruckes zu einer Gewährleistung der gerichteten Strömung im Reaktor führt. Die Schrift offenbart nicht, unter welchem Druck die Verdampfung des Amins durchgeführt wird.

Für den Gesamtdruck im Reaktionsraum der Umsetzung von Phosgen und Diamin offenbart die WO 2008/055898 A1 einen Bereich von Absolutdrücken von 0,1 bis weniger als 20 bar, bevorzugt 0,5 und 15 bar, und besonders bevorzugt zwischen 0,7 und 10 bar. Dabei ist der Druck unmittelbar vor der Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 2000 mbar, besonders bevorzugt von 30 bis 1000 mbar höher als im Reaktionsraum. Die Schrift offenbart nicht, unter welchem Druck die Verdampfung des Amins durchgeführt wird.

Neben den Reaktionsbedingungen ist auch die Art und Weise der Verdampfung der in der Umsetzung mit Phosgen in der Gasphase eingesetzten Amine Gegenstand offenbarter Verfahren.

Gemäß dem obigen Stand der Technik zum Zwecke der Gasphasenphosgenierung werden die Ausgangsamine verdampft und auf 200 bis 600°C erhitzt, bevor sie zusammen mit Phosgen in einen Reaktionsraum geführt werden, in dem ein Druck von 0,1 bis weniger als 20 bar herrscht. Diese im Stand der Technik offenbarten Reaktionsbedingungen umspannen einen weiten Bereich und geben keine konkreten Hinweise, unter welchen Bedingungen das Ausgangsamin verdampft werden sollte, da insbesondere nicht alle Kombinationen aus den genannten Bereichen zur Verdampfung von Aminen geeignet sind, da viele für die Gasphasenphosgenierung geeigneten Amine z.B. bei 20 bar und 200°C nicht verdampfbar sind.

Einzelne Hinweise, unter welchen Bedingungen das Ausgangsamin verdampft werden sollte, betreffen die thermische Belastung des Amins und den Gehalt an Flüssigkeitstropfen im verdampften Aminstrom.

Der thermischen Belastung der in der Gasphasenphosgenierung eingesetzten Amine nimmt sich EP 1 754 698 A1 durch die Beanspruchung einer speziellen Verdampfertechnik an. Nach der Lehre von EP 1 754 698 A1 werden die in dem Reaktor zur Umsetzung der eingesetzten Amine mit Phosgen zu beobachtenden Ablagerungen zum Ersten durch die Zersetzung der eingesetzten Amine während der Reaktion verursacht. Zum Zweiten führen lange Verweilzeiten in der Verdampfung und Überhitzung zu einer partiellen Zersetzung der eingesetzten Amine unter Ammoniak-Abspaltung. Diese speziell bei Einsatz von aliphatischen Aminen zu beobachtende partielle Zersetzung unter Ammoniak-Abspaltung während der Verdampfung vermindert nicht nur die Ausbeute, sondern es bilden sich bei der nachfolgenden Phosgenierungsreaktion und in den nachgeschalteten Rohrleitungen und Apparaten auch Ablagerungen von Ammoniumchlorid. Die Anlagen müssen dann relativ häufig gereinigt werden, wobei entsprechende Produktionsverluste entstehen. Als technische Lösung offenbart diese Schrift die Ammoniak-Abspaltung während der Verdampfung dadurch zu unterdrücken, dass für die Verdampfung und Überhitzung der aliphatischen Amine spezielle Milli- oder Mikrowärmetauscher eingesetzt werden sollten. Die Schrift offenbart, dass durch den Einsatz der offenbarten Wärmetauscher mit einer sehr hohen volumenspezifischen Wärmetauscheroberfläche durch verringerte Filmdicken die thermische Belastung der Amine in den Verdampfungsapparaturen verringert werden kann. Dabei können in diesen Apparaten jeweils bei Drücken von 500 bis 2500 mbar die Erhitzung, die Verdampfung und die Überhitzung des Amins jeweils innerhalb von Verweilzeiten von 0,001 bis 60 s bzw. 0,0001 bis 10 s erfolgen. Kriterien für die Überhitzung des Diamins und innerhalb welcher Zeit das dampfförmige Diamin dem Reaktionsraum zugeführt wird, werden nicht genannt, da der Schrift lediglich Verweilzeiten für einzelne Apparate zu entnehmen sind.

Nachteilig an den in der Schrift EP 1 754 698 A1 offenbarten Mikrowärmetauschern sind die sehr kleinen Kanäle, so dass bereits sehr kleine Feststoffmengen, die in technischen Prozessen stets vorhanden sind, zu einer Verstopfung führen, und somit die Standzeit der Verdampfers reduzieren. Weiterhin nachteilig an der offenbarten Totalverdampfung der Amine ist, dass die Amine keine nicht verdampfbaren Bestandteile enthalten dürfen, weil sich diese sonst zwangsläufig als fester Rückstand auf der Verdampferoberfläche ablagern, damit den Wärmeübergang verschlechtern und schließlich zur Verstopfung des Verdampfers führen. Die Bereitstellung von Aminen in der geforderten Qualität ist im technischen Prozess jedoch sehr aufwendig und teuer. Somit wird durch die Lehre dieser Schrift zwar die Standzeit des Reaktors verbessert, die Standzeit des Verdampfersystems jedoch deutlich verschlechtert, so dass die gesamte Standzeit der Produktionsanlage nicht vorteilhaft verbessert wird.

Zur Beurteilung eines durch Verdampfung erhaltenen Stroms unterscheidet der Fachmann generell zwischen Dampf und Gas. Weiterhin unterscheidet der Fachmann in diesem Zusammenhang zwischen Siedetemperatur und Tautemperatur. Eine Flüssigkeit beginnt bei der Erhitzung bis an den Siedepunkt und durch zusätzliche Zuführung der zur Verdampfung notwendigen Energie zu verdampfen. Man erhält dabei eine Dampfphase oberhalb der Flüssigkeit, die mit dieser im Gleichgewicht steht. Dabei befindet sich die oberhalb der siedenden Flüssigkeit erhaltene Dampfphase am Taupunkt, d.h. der Temperatur, bei der die Kondensation des Dampfes unter Bildung von Flüssigkeit beginnt. Sofern z.B. eine kalte Stelle vorhanden ist oder sich die Temperatur des Dampfes nur geringfügig abkühlt, wird damit der Dampf kondensieren. Kennzeichen eines dampfförmigen Stroms für den Fachmann ist also, dass bereits eine geringfügige Temperaturerniedrigung - wie sie z.B. durch Wärmebrücken oder eine nicht perfekte Isolierung im technischen Maßstab nie ganz zu vermeiden ist - zu einer Kondensation und damit zur Ausbildung einer flüssigen Phase führt.

Ein Gas hingegen ist dadurch gekennzeichnet, das bei der Abkühlung eines Gases sich zunächst die Gastemperatur nur verringert, ohne dass dabei die Tautemperatur unterschritten wird und ohne dass es somit zu einer Kondensation und Ausbildung einer flüssigen Phase kommt. Erst wenn die Gastemperatur weiter bis auf die Tautemperatur erniedrigt wird, beginnt die Kondensation, es entsteht eine flüssige Phase und der Dampfbereich ist erreicht.

In technischen Verdampfungsapparaturen kommt es neben dem allgemein gültigen FlüssigkeitsDampf-Gleichgewicht noch zu dem Effekt, dass Flüssigkeitstropfen durch die Wirkung des verdampfenden Materials aus der Flüssigkeit herausgerissen werden, d.h. Tröpfchen mitgerissen werden und sich dadurch ebenfalls zusätzlich in der Dampfphase befinden. Im Allgemeinen haben die mitgerissenen Tröpfchen eine Größe von bis zu 1000 µm. Durch diesen Effekt kann somit auch eine oberhalb der Tautemperatur befindliche Gasphase Tröpfchen enthalten. Dies ist insbesondere dort zu beobachten, wo geschlossene Flüssigkeitsfilme aufreisen, z.B. am Boden von Fallfilmverdampfern oder beim Blasensieden innerhalb von Kesselverdampfern.

Der Tropfengehalt im dampfförmigen Diaminstrom war bereits Gegenstand früherer Überlegungen: Gemäß EP 1 935 876 A1 sollen die dampfförmigen Amine im Wesentlichen keine Tröpfchen an unverdampften Aminen enthalten, das heißt maximal 0,5 Gew.-% des Amins, und insbesondere maximal 0,05 Gew.-% des Amins, bezogen auf das Gesamtgewicht des Amins, liegen in der Form von unverdampften Tröpfchen vor und der restliche Teil des Amins liegt dampfförmig vor. Ganz besonders bevorzugt sollen nach der Lehre der EP 1 935 876 A1 die dampfförmigen Amine keine Tröpfchen an unverdampften Aminen enthalten. Durch die Erzeugung eines im Wesentlichen tropfenfreien dampfförmigen Diaminstroms vor Eintritt in den Reaktor wird gemäß EP 1 935 876 A1 die Reaktorlaufzeit deutlich erhöht. Nach Lehre dieser Schrift kann dieses erreicht werden, in dem zwischen den Verdampfungs- und Überhitzungssystemen Tropfenabscheider eingebaut sind und / oder die Verdampfungsapparaturen auch die Funktion eines Tropfenabscheiders besitzen. Geeignete Tropfenabscheider sind z.B. beschrieben in "Droplet Separation", A. Bürkholz, VCH Verlagsgesellschaft, Weinheim - New York - Basel - Cambridge, 1989. Als besonders bevorzugt nennt EP 1 935 876 A1 Tropfenabscheider, die einen geringen Druckverlust verursachen. Lehre dieser Schrift für den Fachmann ist somit, dass sich an die Verdampfung des Amins unter Erhalt eines dampfförmigen Aminstromes ein Tropfenabscheider mit möglichst geringem Druckverlust anschließt, um eine lange Standzeit der Gasphasenreaktion zu erreichen.

Das offenbarte Verfahren ist jedoch insbesondere für die großtechnische Durchführung der Gasphasenphosgenierung nachteilig, da der Druckverlust von Tropfenabscheidern mit dem Volumenstrom sehr stark ansteigt. Zudem ist der Abscheidegrad, d.h. der Anteil der durch den Tropfenabscheider abgeschiedenen Tröpfchen, bei einem hohen Volumenstrom kleiner als bei niedrigem Volumenstrom bei gleichem Druckverlust. Somit müssten zur großtechnischen Durchführung entsprechend der Lehre von EP 1 935 876 A1 entweder Tropfenabscheider mit einem geringen Abscheidegrad verwendet werden, um den durch den Abscheider verursachten Druckverlust zu minimieren. Damit wäre der Dampfstrom nicht mehr tropfenfrei - mit den bekannten Nachteilen für die Standzeit der Gasphasenreaktion. Oder es müssten Tropfenabscheider mit einem hohen Abscheidegrad verwendet werden, um den nach Lehre des Stands der Technik notwendigen tropfenfreien Aminstrom zu erzeugen, die jedoch einen hohen Druckverlust verursachen.

Ein erhöhter Druckverlust im Strom des dampfförmigen Amins bei der großtechnischen Durchführung ist jedoch gemäß EP 1 754 698 B1 nachteilig, da durch die Druckerhöhung im Verdampfer die Siedetemperatur ansteigt und die Erhöhung der für die Verdampfung erforderliche Temperatur eine stärkere Abspaltung von Ammoniak aus dem Diamin bewirken kann, der wiederum durch die Bildung von Ammoniumchlorid zur Verstopfung des Gasphasenreaktors führt und somit die Standzeit der Gasphasenreaktion verkürzt.

Ferner ist ein hoher Druckverlust auch nachteilig, weil eine hohe Druckdifferenz zu einer höheren Gasgeschwindigkeit des dampfförmigen, tropfenhaltigen Diaminstroms führt, wobei eine hohe Gasgeschwindigkeit eines mit Flüssigkeitstropfen beladenen Gasstroms nachteilig ist, da es dadurch zu Abrasion an den Rohrleitungen kommen kann.

Der Fachmann entnimmt somit dem Stand der Technik zur Gasphasenphosgenierung die allgemeine Lehre, dass die Verdampfung von Diaminen einen tropfenhaltigen Dampfstrom ergibt, der über druckverlustverursachende Tropfenabscheider geführt werden muss, um so eine vorteilhafte Tropfenfreiheit zu erreichen, wobei gleichzeitig die mit dem Druckverlust verbundenen Nachteile (Siedepunktserhöhung des Diamins, Abrasion in den Rohrleitungen) technisch gelöst werden müssen. Bei der praktischen Durchführung des Verfahrens im großtechnischen Maßstab kann nach dem Stand der Technik die Erzeugung eines im Wesentlichen tropfenfreien Aminstroms nur unter Inkaufnahme eines hohen Druckverlustes zwischen Aminverdampfung und Reaktion erfolgen, wodurch unausweichlich die aus dem Stand der Technik negativen Konsequenzen für die Standzeit einer Gasphasenreaktion folgen, da ein hoher Druckverlust zwischen Reaktor und Aminverdampfung einen hohen Verdampfungsdruck und damit eine erhöhte Verdampfungstemperatur bedeutet. Keine der bisher offenbarten Schriften befasst sich mit diesen Problemen; daher ist dem Stand der Technik eine Lösung derselben nicht zu entnehmen.

Aufgabe der Erfindung war es daher, ein Verfahren zur großtechnischen Herstellung von Isocyanaten durch Phosgenierung in der Gasphase bereitzustellen, das sich dadurch auszeichnet, dass der zum Reaktor geführte Aminstrom weitestgehend tropfenfrei ist und gleichzeitig die Druckdifferenz zwischen Aminverdampfung und Reaktor gering ist.

Überraschend wurde nun gefunden, dass eine für die Gasphasenphosgenierung im großtechnischen Maßstab vorteilhafte Tropfenfreiheit im Diaminstrom auch dann erreicht wird, wenn der Druck im Verdampfer lediglich höchstens 500 mbar höher als im Reaktor ist, und gleichzeitig der den Verdampfer verlassende Diaminstrom mit einer mittleren Verweilzeit von mehr als 0,03 s dem Reaktor zur Umsetzung so zugeführt wird, dass der Diaminstrom am Eintritt in den Reaktor eine Temperatur von mindestens 15 °C über dem Taupunkt hat.

Die Beschränkung des Druckverlustes zwischen Verdampfer und Reaktor schränkt die Auswahl der möglichen Apparate zur Tropfenabscheidung ein, bringt aber die Vorteile eines niedrigen Diamin-Siedepunktes und niedriger Gasgeschwindigkeiten mit sich. In einer möglichen Ausführungsform des erfindungsgemäßen Verfahrens wird ganz auf den Einsatz von Apparaten zur Tropfenabscheidung verzichtet.

Die damit verbundenen Einschränkungen bei der Tropfenabscheidung können aufgrund der erfinderischen neuen Verfahrensführung in Kauf genommen werden.

Es wurde überraschenderweise gefunden, dass die Abscheidung von Tröpfchen aus dem Diaminstrom nicht in vollem Umfange durch druckverlustverursachende Tropfenabscheider erfolgen muss. Vielmehr kann auch ohne vollständige Tropfenabscheidung, die einen hohen Druckverlust verursachen würde, eine lange Reaktorstandzeit erzielt werden, sofern Verweilzeit und Temperaturerhöhung über den Taupunkt des Diaminstromes vor Eintritt in den Reaktor ausreichen, um die nach der Verdampfung im Dampfstrom vorhandenen Tröpfchen bis zum Eintritt in den Reaktor vollständig zu verdampfen. Durch die Erfindung wird somit ein Verfahren zur Verfügung gestellt, bei dem ein weitestgehend tropfenfreier Aminstrom zum Reaktor geleitet wird und zudem aufgrund der geringen Druckdifferenz zwischen Reaktor und Aminverdampfung eine niedrige Aminsiedetemperatur erhalten wird. Somit löst die vorliegende Erfindung das Problem, dass für eine lange Laufzeit einer Gasphasenreaktion sowohl eine niedrige Aminsiedetemperatur als auch ein weitestgehend tropfenfreier Aminstrom zum Reaktor notwendig ist.

Gegenstand der Erfindung ist daher ein Verfahren zur Herstellung von Isocyanaten durch Umsetzung von primären Aminen mit Phosgen in der Gasphase, bei dem
a) das primäre Amin in einem Verdampfer verdampft wird, und
b) das in Schritt a) erhaltene verdampfte Amin, das noch Tröpfchen enthält, aus dem Verdampfer austritt und über eine Zuführung, in welcher ein Überhitzer angeordnet ist, zum Reaktor geleitet wird und in den Reaktor eingeführt wird,
dadurch gekennzeichnet dass
c) das in Schritt a) erhaltene verdampfte Amin, das noch Tröpfchen enthält, in der Zuführung zum Reaktor überhitzt wird, so dass es in der Zuführung unmittelbar vor Eintritt in den Reaktor eine Temperatur von mindestens 15 °C über dem Taupunkt aufweist, wobei in der Zuführung eine Einrichtung zur Tropfenabscheidung angeordnet ist, die einen Grenztropfendurchmesser von 5 bis 550 µm aufweist, wobei diese Einrichtung zur Tropfenabscheidung keine vollumfängliche Tropfenabscheidung bewirkt und
d) die Verweilzeit des verdampften Amins in Schritt b) mehr als 0,03 Sekunden beträgt, und
e) die Druckdifferenz über die Zuführung zwischen dem Austritt aus dem Verdampfer und dem

Eintritt in den Reaktor zwischen 1 und 500 mbar beträgt.

In einer bevorzugten Ausführung der vorliegenden Erfindung wird das Diamin zum Zwecke der Gasphasenphosgenierung in mindestens einem Verdampfer verdampft sowie auf Temperaturen von 200 bis 600°C, bevorzugt 200 bis 500°C, besonders bevorzugt 250 bis 450°C erhitzt und gegebenenfalls verdünnt mit einem Inertgas wie N₂, He, Ar oder mit den Dämpfen eines inerten Lösungsmittels, z.B. aromatischen Kohlenwasserstoffen ggf. mit Halogensubstitution, wie z.B. Chlorbenzol oder ortho-Dichlorbenzol, dem Reaktionsraum zugeführt.

Als Verdampfer für die Aminverdampfung können grundsätzlich beliebige geeignete Verdampfer eingesetzt werden. Bevorzugt können Rohrbündelwärmetauscher, Plattenwärmetauscher oder Fallfilmverdampfer, gegebenenfalls mit Umpumpkreislauf, eingesetzt werden. Auch können Mikrowärmetauscher oder Mikroverdampfer wie in WO 2005/016512 A oder in DE 10 2005 036870 A1 beschrieben eingesetzt werden.

Bevorzugt werden Verdampfungssysteme eingesetzt, bei denen ein kleiner Arbeitsinhalt mit einer hohen Umwälzleistung über einen Fallfilmverdampfer geführt wird. Zur Minimierung der thermischen Belastung der Diamine wird der Verdampfungsvorgang - wie oben ausgeführt - ggf. unter Zuspeisung von Inertgas und / oder Lösungsmitteldämpfen unterstützt.

Bei dem erfindungsgemäßen Verfahren kann sowohl die Verdampfung als auch die Überhitzung einstufig erfolgen, sie kann aber auch jeweils mehrstufig erfolgen. Der dampfförmige Diaminstrom wird nach dem ersten bzw. ggfs. einzigen Überhitzer oder alternativ nach dem Verdampfer durch einen Tropfenabscheider mit einem Grenztropfendurchmesser von 5 bis 550 µm, bevorzugt 10 bis 100 µm, geführt. Die Tropfenabscheider weisen bevorzugt einen niedrigen Druckverlust, aber maximal einen solchen Druckverlust auf, dass der Druckverlust zwischen dem Austritt aus dem Verdampfer und dem Eintritt in den Reaktor zwischen 1 und 500 mbar beträgt.

Erfindungsgemäß wird der aus der Verdampfung erhaltende Dampfstrom nach der Verdampfung in der Zuführung zum Reaktor überhitzt, so dass das Gas unmittelbar vor Eintritt in den Reaktor eine Temperatur von mindestens 15°C und bevorzugt von mindestens 25°C oberhalb des Taupunkts aufweist. Durch den Transport der nicht abgeschiedenen Flüssigkeitstropfen in dem so überhitzten Diaminstrom nehmen die Tröpfchen Energie aus dem umgebenen heißen Gas auf und verdampfen. Die Art der Überhitzung ist nicht erfindungswesentlich, sie kann z.B. durch einen Apparat wie z.B. einem Rohrbündelwärmetauscher oder durch eine beheizte Rohrleitung erfolgen. In einer speziellen Ausführungsform fungiert der Überhitzer mit dem das verdampfte Amin auf eine Temperatur von mindestens 15°C, bevorzugt 25°C oberhalb des Taupunktes des Diamins überhitzt wird, auch als Tropfenabscheider. In einer besonders speziellen Ausführungsform hat dieser Überhitzer einen Flüssigkeitsablauf um die stetige Entleerung des Tropfenabscheiders zu gewährleisten.

Bevorzugt wird die Verdampfung in Schritt a) bei absoluten Drücken von zwischen 0,1 und 20 bar durchgeführt.

Die mittlere Verweilzeit vom Verlassen des Verdampfers bis zum Eintritt in den Reaktor beträgt mehr als 0,03 Sekunden, bevorzugt mehr als 0,1 und insbesondere bevorzugt mehr als 0,5 Sekunden. Im Allgemeinen beträgt die Verweilzeit bis zum Eintritt in den Reaktor nicht mehr als 60 s. Dabei wird über technische Maßnahmen, z.B. eine ausreichende Isolierung zur Vermeidung von Abstrahlungsverlusten, oder einer Begleitbeheizung der Rohrleitung mittels Wärmeträgeröl, Rauchgas oder elektrischer Begleitbeheizung, verhindert, dass das sich Gas bei Eintritt in den Reaktor unter die erfindungsgemäße Temperaturgrenze zur Tautemperatur abkühlt.

Überraschenderweise wurde gefunden, dass durch die Kombination der Überhitzung des Gases um mindestens 15°C und bevorzugt um mindestens 25°C über den Taupunkt vor Eintritt in den Reaktor in Kombination mit einer Verweilzeit von mehr als 0,03 und bevorzugt mehr als 0,08 Sekunden in dem Strom, der aus der Verdampfung erhalten wird und noch Tröpfchen enthält, ein weitestgehend tropfenfreier Amingasstrom zum Reaktor erhalten werden kann.

Durch das erfindungsgemäße Verfahren kann die Druckdifferenz zwischen dem Eintritt in den Reaktor und der Aminverdampfung auf nicht mehr als 500 mbar begrenzt werden. Im allgemeinen ist eine Druckdifferenz von mehr als 1 mbar notwendig, bevorzugt mehr als 10 mbar und insbesondere bevorzugt von mehr als 20 mbar um eine ausreichende Strömungsgeschwindigkeit des Gases zu erhalten. Bevorzugt beträgt die Druckdifferenz zwischen dem Eintritt in den Reaktor und der Aminverdampfung nicht mehr als 450 mbar, insbesondere bevorzugt nicht mehr als 400 mbar.

Dies bedeutet, dass der Druck in der Aminverdampfung nicht mehr als 500 mbar oberhalb des Druckes am Eintritt des Reaktors ist. Dadurch wird die Siedetemperatur des Amins niedrig gehalten und es können sich die aus dem Stand der Technik beschriebenen Nachteile aus der Aminzersetzung bei erhöhter Siedetemperatur vermeiden lassen. Somit wirkt sich die geringe Druckdifferenz positiv auf die Standzeit der Gasphasenreaktion aus.

Durch die Einhaltung der oben beschriebenen Anforderungen an die Verweilzeit in der Zuführung gemäß Schritt b) und Überhitzung wird bei geringem Druckverlust und damit geringer Verdampfungstemperatur ein im Wesentlichen tropfenfreier dampfförmiger Diaminstrom vor Eintritt in den Reaktor erhalten und dadurch die Reaktorlaufzeit deutlich erhöht. Unter einem im Wesentlichen tropfenfreien dampfförmigen Ausgangsaminstrom wird verstanden, dass die dampfförmigen Amine im Wesentlichen keine Tröpfchen an unverdampften Aminen, das heißt, dass maximal 0,5 Gew.-% des Amins, besonders bevorzugt maximal 0,05 Gew.-% des Amins, bezogen auf das Gesamtgewicht an Amin, in der Form von unverdampften Tröpfchen vorliegt und der restliche Teil des Amins dampfförmig vorliegt. Ganz besonders bevorzugt enthalten die dampfförmigen Amine keine Tröpfchen an unverdampften Aminen.

Die Verdampfer und / oder Überhitzer sowie die Rohrleitungen zur Erzeugung des dampfförmigen Diaminstroms zum Gasphasenreaktor können aus einem beliebigen metallischen Werkstoff gefertigt sein, z.B. Stahl, Edelstahl, Titan, Hastelloy, Inconel oder anderen metallischen Legierungen. Bevorzugt werden metallische Werkstoffe mit einem geringen Nickel-Anteil verwendet. Ferner weisen die Rohrleitungen bevorzugt auf ihrer ganzen Länge, jedoch mindestens teilweise ein Gefälle zum Sumpf des Verdampfers hin auf, so dass ggf. anfallendes Kondensat in den Verdampfer zurückfließen kann.

Die erfindungsgemäße Umsetzung des primären Amines mit Phosgen erfolgt in einem Reaktor. Der Reaktor enthält zumindest einen Reaktionsraum. Unter Reaktionsraum wird der Raum verstanden, in dem die Vermischung und die Umsetzung der Edukte und Zwischenprodukte erfolgen. Denn aufgrund der hohen Geschwindigkeit der Gasphasenreaktion lassen sich Vermischung und Umsetzung in dem erfindungsgemäßen Verfahren kaum räumlich trennen. Unter Reaktor wird die technische Vorrichtung verstanden, die den Reaktionsraum enthält. Ein Reaktor kann dabei auch mehrere Reaktionsräume enthalten.

Das erfindungsgemäße Verfahren ist prinzipiell auf jede Reaktionsraum- und Reaktorgeometrie anwendbar.

In einer weiteren bevorzugten Ausführungsform des erfindungsgemäßen Verfahrens weist der Reaktor im Anschluss an den Reaktionsraum, in dem nach der Mischung der Edukte ein Umsatz der Amingruppen zu den Isocyanatgruppen von 80 %, bevorzugt 90 %, besonders bevorzugt 99 %, ganz besonders bevorzugt 99,5 % erzielt wird, einen rotationssymmetrischen Reaktionsraum mit konstanter und / oder erweiterter durchströmter Querschnittsfläche auf.

Das erfindungsgemäße Verfahren ist prinzipiell auf jede Fahrweise der Gasphasenphosgenierung anwendbar. Bevorzugt ist die in EP 1 935 876 A1 beschriebene adiabate Fahrweise. Das beschriebene Verfahren ist jedoch auch in einer isothermen Fahrweise anwendbar.

Die gewählte Verweilzeit für die Reaktion der Amingruppen mit dem Phosgen zu dem Isocyanat liegt zwischen 0,05 und 15 Sekunden, in Abhängigkeit von der Art des eingesetzten Amins, der Starttemperatur, ggf. der adiabaten Temperaturerhöhung im Reaktionsraum, dem molaren Verhältnis von eingesetztem Amin und Phosgen, Art und Menge des mindestens einen inerten Stoffes sowie dem gewählten Reaktionsdruck.

Beim erfindungsgemäßen Verfahren ist es vorteilhaft, Phosgen im Überschuss bezüglich der umzusetzenden Amingruppen einzusetzen. Bevorzugt liegt ein molares Verhältnis von Phosgen zu Amingruppen von 1,1:1 bis 20:1, bevorzugt 1,2:1 bis 5:1 vor. Auch das Phosgen wird auf Temperaturen von 200 bis 600°C erhitzt und gegebenenfalls verdünnt mit einem Inertgas wie N₂, He, Ar oder mit den Dämpfen eines inerten Lösungsmittels, z.B. aromatischen Kohlenwasserstoffen ohne oder mit Halogensubstitution, wie z.B. Chlorbenzol oder o-Dichlorbenzol, dem Reaktionsraum zugeführt.

Nach der erfolgten Phosgenierungsreaktion im Reaktionsraum wird bevorzugt das gasförmige Reaktionsgemisch, das bevorzugt wenigstens ein Isocyanat, Phosgen, ggfs einen inerten Stoff sowie Chlorwasserstoff umfasst, von dem gebildeten Isocyanat befreit. Dies kann beispielsweise so erfolgen, dass das den Reaktionsraum kontinuierlich verlassende Reaktionsgemisch, einer Kondensation in einem inerten Lösungsmittel unterzogen wird, wie sie bereits für andere Gasphasenphosgenierungen empfohlen wurde (EP 0 749 958 A1).

Bevorzugt erfolgt die Kondensation jedoch dadurch, dass der in dem erfindungsgemäßen Verfahren eingesetzte Reaktionsraum zumindest eine Zone aufweist, in die zum Abbruch der Umsetzung der eingesetzten Amine und des Phosgens zu den entsprechenden Isocyanaten ein oder mehrere geeignete Flüssigkeitsströme ("Quench-Flüssigkeiten") eingesprüht werden. Dadurch kann, wie in EP 1 403 248 A1 beschrieben, eine rasche Abkühlung der Gasgemische ohne den Einsatz kalter Flächen durchgeführt werden.

In einer besonders bevorzugten Form des erfindungsgemäßen Verfahrens ist die zumindest eine Zone (Kühlzone) in eine Quenchstufe, wie sie z. B. in EP 1 403 248 A1 offenbart wurde, integriert. In einer besonders bevorzugten Form kommen mehrere Kühlzonen zum Einsatz. Integration und Betrieb dieser zumindest zwei Kühlzonen erfolgen bevorzugt mit einer Quenchstufe. Dies ist hinsichtlich Aufbau und Betrieb in EP 1 935 875 A1 offenbart.

Statt des integrierten Verbundes von der zumindest einen Kühlzone eines Reaktors mit einer Quenchstufe, wie sie in der EP 1 935 875 A1 offenbart wurde, ist ebenfalls der entsprechende integrierte Verbund der Kühlzonen mehrerer Reaktoren mit einer Quenchstufe möglich. Bevorzugt ist jedoch der integrierte Verbund eines Reaktors mit zumindest einer Kühlzone mit einer Quenchstufe.

Das die Kondensations- bzw. Quenchstufe verlassende Gasgemisch wird bevorzugt in einer nachgeschalteten Gaswäsche mit einer geeigneten Waschflüssigkeit von restlichem Isocyanat befreit, bevorzugt anschließend in an sich bekannter Weise von überschüssigem Phosgen befreit. Dies kann mittels einer Kühlfalle, Absorption in einem inerten Lösungsmittel (z. B. Chlorbenzol oder Dichlorbenzol) oder durch Adsorption und Hydrolyse an Aktivkohle erfolgen. Das die Phosgenrückgewinnungsstufe durchlaufende Chlorwasserstoffgas kann in an sich bekannter Weise zur Rückgewinnung des zur Phosgensynthese erforderlichen Chlors recyclisiert werden. Die nach ihrem Einsatz zur Gaswäsche anfallende Waschflüssigkeit wird dann bevorzugt zumindest teilweise als Quench-Flüssigkeit zur Abkühlung des Gasgemisches in die entsprechende Zone des Reaktionraumes eingesetzt.
Die Reindarstellung der Isocyanate erfolgt bevorzugt anschließend durch destillative Aufarbeitung der Lösungen bzw. Gemische aus der Kondensations- bzw. Quenchstufe.
In dem erfindungsgemäßen Verfahren werden bevorzugt solche primären Amine und insbesondere solche Diamine eingesetzt, die im Wesentlichen ohne Zersetzung in die Gasphase überführt werden können.

Beispiele für bevorzugte aliphatische bzw. cycloaliphatische Diamine sind 1,4-Diaminobutan, 1,6-Diaminohexan (HDA), 1,11-Diaminoundecan, 1-Amino-3,5,5-trimethyl-3-aminomethylcyclohexan (IPDA), 4,4'-Diaminodicyclohexylmethan oder 4,4'-Diaminodicyclohexylpropan-2,2. Besonders bevorzugt sind jedoch Diamine der vorstehend genannten Art mit ausschließlich aliphatisch oder cycloaliphatisch gebundenen Aminogruppen wie Isophorondiamin (IPDA), Hexamethylendiamin (HDA) oder Bis(p-aminocyclohexyl)methan (PACM 20).
Beispiele für bevorzugte aromatische Diamine sind Toluylendiamin (TDA), insbesondere 2,4-TDA und 2,6-TDA und Gemische daraus, Diaminobenzol, Naphthyldiamin (NDA) und 2,2'-, 2,4'-oder 4,4'-Methylendiphenyldiamin (MDA) oder Isomerengemische davon. Besonders bevorzugt ist Toluylendiamin (TDA), insbesondere 2,4-TDA und 2,6-TDA und Gemische daraus. Ganz besonders bevorzugt sind 2,4- / 2,6-TDA-Isomerengemische der Isomerenverhältnisse 80/20 und 65/35.

### Beispiele

### A. Erfindungsgemäßes Beispiel

Ein TDA-Strom von 2500 kg/h wird bei einer Temperatur von 315°C verdampft, durch einen Tropfenabscheider mit einem Grenztropfendurchmesser von 530 µm geführt und dem Reaktor zugeführt. Bei Austritt aus dem Tropfenabscheider enthält der Strom 10 Gew.-% unverdampfte Tröpfchen. Die Zuführung zum Reaktor erfolgt so, dass der Strom nach Austritt aus der Verdampfung überhitzt wird, wobei der Strom bei Eintritt in den Reaktor um 15°C über den Taupunkt erhitzt ist. Die Verweilzeit des Gases in der Zuführung zum Reaktor beträgt 4,1 Sekunden. Dabei verdampfen die zunächst vorhandenen Tröpfchen, so dass der Strom zum Reaktor im Wesentlichen tropfenfrei ist. Die Druckdifferenz zwischen Verdampfung und Eintritt in den Reaktor beträgt weniger als 500 mbar.

### B. Erfindungsgemäßes Beispiel

Ein TDA-Strom von 2500 kg/h wird bei einer Temperatur von 315°C verdampft, durch einen Tropfenabscheider mit einem Grenztropfendurchmesser von 100 µm geführt und dem Reaktor zugeführt. Bei Austritt aus dem Tropfenabscheider enthält der Strom noch 10 Gew.-% unverdampfte Tröpfchen. Die Zuführung zum Reaktor erfolgt so, dass der Strom nach Austritt aus der Verdampfung überhitzt wird, wobei der Strom bei Eintritt in den Reaktor um 15°C über den Taupunkt erhitzt ist. Die Verweilzeit des Gases in der Zuführung zum Reaktor beträgt 0,7 Sekunden. Dabei verdampfen die zunächst vorhandenen Tröpfchen, so dass der Strom zum Reaktor im Wesentlichen tropfenfrei ist. Die Druckdifferenz zwischen Verdampfung und Eintritt in den Reaktor beträgt weniger als 500 mbar.

## Patentansprüche

1. Verfahren zur Herstellung von Isocyanaten durch Umsetzung von primären Aminen mit Phosgen in der Gasphase, bei dem
a) das primäre Amin in einem Verdampfer verdampft wird, und
b) das in Schritt a) erhaltene verdampfte Amin, das noch Tröpfchen enthält, aus dem Verdampfer austritt und über eine Zuführung, in welcher ein Überhitzer angeordnet ist, zum Reaktor geleitet wird und in den Reaktor eingeführt wird,
**dadurch gekennzeichnet dass**
c) das in Schritt a) erhaltene verdampfte Amin, das noch Tröpfchen enthält, in der Zuführung zum Reaktor überhitzt wird, so dass es in der Zuführung unmittelbar vor Eintritt in den Reaktor eine Temperatur von mindestens 15°C über dem Taupunkt aufweist,
wobei in der Zuführung eine Einrichtung zur Tropfenabscheidung angeordnet ist, die einen Grenztropfendurchmesser von 5 bis 550 µm aufweist, wobei diese Einrichtung zur Tropfenabscheidung keine vollumfängliche Tropfenabscheidung bewirkt,
und
d) die Verweilzeit des verdampften Amins in Schritt b) mehr als 0,03 Sekunden beträgt, und
e) die Druckdifferenz über die Zuführung zwischen dem Austritt aus dem Verdampfer und dem Eintritt in den Reaktor zwischen 1 und 500 mbar beträgt.

2. Verfahren nach Anspruch 1, bei dem das eingesetzte Amin Toluylendiamin, Phenyldiamin, Naphthyldiamin, Methylendiphenyldiamin, Hexamethylendiamin und / oder Isophorondiamin ist.

3. Verfahren nach Anspruch 1, bei dem die Verweilzeit des verdampften Amins in Schritt b) mehr als 0,1 Sekunden beträgt.

4. Verfahren nach Anspruch 1, bei dem die Einrichtung zur Tropfenabscheidung einen Flüssigkeitsablauf aufweist, aus dem kontinuierlich oder in periodischen Abständen Flüssigkeit entnommen werden kann.

5. Verfahren nach Anspruch 1, bei dem die Verdampfung des Amins in Gegenwart eines Inertgases und / oder in Gegenwart der Dämpfe eines inerten Lösungsmittels durchgeführt wird.

6. Verfahren nach Anspruch 1, bei dem die Verdampfung in Schritt a) bei einem absoluten Druck von zwischen 0,1 und 20 bar durchgeführt wird.

7. Verfahren nach Anspruch 1, bei dem das verdampfte Amin im Überhitzer in der Zuführung in Schritt b) auf eine Temperatur von 200 bis 600°C erhitzt wird.

## Claims

1. Process for the preparation of isocyanates by reaction of primary amines with phosgene in the gas phase, in which
a) the primary amine is vaporized in a vaporizer, and
b) the vaporized amine obtained in step a), which still contains droplets, exits from the vaporizer and is led to the reactor via a feed, in which a superheater is arranged, and is introduced into the reactor,
**characterized in that**
c) the vaporized amine obtained in step a), which still contains droplets, is superheated in the feed to the reactor, so that it has a temperature of at least 15 ºC above the dew point in the feed directly before entry into the reactor,
wherein a device for drop separation which has a drop diameter limit of from 5 to 550 µm is arranged in the feed, where this device for drop separation does not bring about comprehensive drop separation, and
d) the dwell time of the vaporized amine in step b) is more than 0.03 second, and
e) the pressure difference over the feed between the exit from the vaporizer and the entry into the reactor is between 1 and 500 mbar.

2. Process according to claim 1, in which the amine employed is tolylenediamine, phenyldiamine, naphthyldiamine, methylenediphenyldiamine, hexamethylenediamine and / or isophoronediamine.

3. Process according to claim 1, in which the dwell time of the vaporized amine in step b) is more than 0.1 second.

4. Process according to claim 1, in which the device for drop separation has a liquid drain from which liquid can be removed continuously or at periodic intervals.

5. Process according to claim 1, in which the vaporization of the amine is carried out in the presence of an inert gas and / or in the presence of the vapours of an inert solvent.

6. Process according to claim 1, in which the vaporization in step a) is carried out under an absolute pressure of between 0.1 and 20 bar.

7. Process according to claim 1, in which the vaporized amine is heated to a temperature of from 200 to 600 ºC in the superheater in the feed in step b).

## Revendications

1. Procédé de fabrication d'isocyanates par mise en réaction d'amines primaires avec du phosgène en phase gazeuse, selon lequel
a) l'amine primaire est évaporée dans un évaporateur, et
b) l'amine évaporée obtenue à l'étape a), qui contient encore des gouttelettes, est déchargée de l'évaporateur et conduite par une canalisation d'alimentation, dans laquelle un dispositif de surchauffage est agencé, dans le réacteur et introduite dans le réacteur,
**caractérisé en ce que**
c) l'amine évaporée obtenue à l'étape a), qui contient encore des gouttelettes, est surchauffée dans la canalisation d'alimentation vers le réacteur, de sorte qu'elle présente dans la canalisation d'alimentation directement avant l'entrée dans le réacteur une température au moins 15 °C au-dessus du point de condensation,
un dispositif pour la séparation des gouttes étant agencé dans la canalisation d'alimentation, qui présente un diamètre de goutte limite de 5 à 550 µm, ce dispositif pour la séparation des gouttes ne réalisant pas une séparation complète des gouttes,
et
d) le temps de séjour de l'amine évaporée dans l'étape b) est supérieur à 0,03 seconde, et
e) la différence de pression dans la canalisation d'alimentation entre la sortie de l'évaporateur et l'entrée dans le réacteur est comprise entre 1 et 500 mbar.

2. Procédé selon la revendication 1, selon lequel l'amine utilisée est la toluylène-diamine, la phényldiamine, la naphtyldiamine, la méthylène-diphényldiamine, l'hexaméthylène-diamine et/ou l'isophorone-diamine.

3. Procédé selon la revendication 1, selon lequel le temps de séjour de l'amine évaporée dans l'étape b) est supérieur à 0,1 seconde.

4. Procédé selon la revendication 1, selon lequel le dispositif pour la séparation des gouttes comprend une sortie de liquide, à partir de laquelle un liquide peut être soutiré en continu ou à intervalles périodiques.

5. Procédé selon la revendication 1, selon lequel l'évaporation de l'amine est réalisée en présence d'un gaz inerte et/ou en présence de la vapeur d'un solvant inerte.

6. Procédé selon la revendication 1, selon lequel l'évaporation à l'étape a) est réalisée à une pression absolue comprise entre 0,1 et 20 bar.

7. Procédé selon la revendication 1, selon lequel l'amine évaporée est portée à une température de 200 à 600 °C dans le dispositif de surchauffage dans la conduite d'alimentation à l'étape b).
